# EUROPÄISCHE PATENTSCHRIFT

(11) **EP 0 733 099 B1**
(45) Veröffentlichungstag und Bekanntmachung des Hinweises auf die Patenterteilung: **27.05.1998**
(21) Anmeldenummer: 95906305.8
(22) Anmeldetag: 05.01.1995
(51) Int. Cl.: C12M 3/06, C12M 3/08, C12N 15/10, C12Q 1/68

(54) **VERFAHREN ZUM ZERKLEINERN VON HOCHMOLEKULAREN STRUKTUREN**
PROCESS FOR REDUCING HIGH-MOLECULAR STRUCTURES
PROCEDE DE FRAGMENTATION DE STRUCTURES DE POIDS MOLECULAIRE ELEVE

(30) Priorität: 07.01.1994 DE 4400255
(43) Veröffentlichungstag der Anmeldung: 25.09.1996
(73) Patentinhaber: QIAGEN GmbH, 40724 Hilden (DE)
(72) Erfinder: BASTIAN, Helge, D-40822 Mettmann (DE)
(74) Vertreter: Meyers, Hans-Wilhelm, Dr.Dipl.-Chem.
(86) Internationale Anmeldenummer: EP9500037
(87) Internationale Veröffentlichungsnummer: WO9518851

(56) Entgegenhaltungen:
- WO-A-92/00132
- WO-A-92/07863
- WO-A-93/11218
- DE-U- 9 112 776
- US-A- 5 114 858

## Beschreibung

Gegenstand der vorliegenden Erfindung ist ein Verfahren zur Zerkleinerung von hochmolekularen Strukturen gemäß Anspruch 1 sowie Verwendungen der Vorrichtung gemäß Anspruch 7.

Ausgangspunkt vieler biologischer Isolationsverfahren sind beispielsweise Zell- und Gewebelysate, die häufig sehr viskose Systeme darstellen. Aufgrund ihrer hohen Viskosität sind diese Systeme kaum weiterbearbeitbar und werden üblicherweise verschiedenen Verfahren zur Homogenisierung oder Verringerung der Viskosität unterzogen.

Insbesondere bei der Isolierung von mRNA ist es notwendig, die diese Substanzen enthaltenden Proben zu homogenisieren mit dem Ziel nicht-viskose Lysate oder Lösungen zu erhalten. Die Herstellung von homogenen Zell-Lysaten oder Lösungen mit geringerer Viskosität ist bei einer Reihe molekularbiologischer Techniken notwendig. Beispielhaft seien im folgenden einige dieser Techniken genannt.

Die direkte Isolierung von Gesamt-RNA oder mRNA aus Zell- oder Gewebelysaten erfordert in den meisten Fällen einen Homogenisierungsschritt, da die Hybridisierungsrate zwischen der zur mRNA Isolierung notwendigen oligo-dT Matrix (z.B. Cellulose, paramagnetische Partikel oder Latexpartikel) in hochviskosen Lösungen, im Gegensatz zu homogenen Lösungen, sehr niedrig ist. Die entsprechenden Matrices können dabei mit der in der Probe ebenfalls vorhandenen hochmolekularen DNA quasi verkleben und eine Reinigung soweit erschweren, daß sie in manchen Fällen sogar unmöglich wird.

Schließlich ist bei der Präparation von Gesamt-RNA, wie z.B. in DE 43 21 904 A1 beschrieben, die zur chromatographischen Aufreinigung notwendige und in eine Apparatur eingesetzte Membran durch hoch-viskose Lysate oder Lösungen schnell verstopft.

Die Protokolle für eine direkte mRNA Isolierung, wie sie im Stand der Technik bekannt sind unter Verwendung bekannter Produkte, gehen davon aus, daß eine Homogenisierung der Proben, die die genannten Substanzen enthalten, unbedingt erforderlich ist.

Es hat sich beispielsweise gezeigt, daß in der Polymerase-Chain-Reaction (PCR) mit genomischer DNA bessere Ergebnisse erzielt werden, wenn gescherte, niedermolekulare DNA eingesetzt wird. Dies könnte damit zusammenhängen, daß kleinere DNA Fragmente leichter denaturierbar sind und so die Hybridisierung des Primers (Primer-Annealing) effizienter ist. In diesem Zusammenhang ist bedeutsam, daß, bei Präparationen von Nucleinsäuren (mRNA, Gesamt-RNA und DNA) für die PCR-Reaktion, Cross-Kontaminationen mit Nucleinsäuren zwischen gleichzeitig oder nacheinander aufgearbeiteten Proben sowie Cross-Kontaminationen mit Nucleinsäuren aus anderen Quellen unbedingt vermieden werden müssen, da die PCR-Reaktion äußerst empfindlich ist, so daß auch Verunreinigungen mit nicht gewünschter Nucleinsäure entsprechend amplifiziert werden würden.

Im Stand der Technik werden zur Homogenisierung bzw. Scherung von Geweben, Zellen und/oder hochmolekularen Stoffen enthaltenden Lösungen im wesentlichen die folgenden Methoden angewandt.

Die Zerkleinerung (Scherung) der hochmolekularen DNA kann durch mechanisches Zerkleinern einer in flüssigen Stickstofftiefgefrorenen Probe mittels Pistill und Mörser erfolgen. Auch durch mehrmaliges Aufziehen der Probe mit hochmolekularer DNA durch eine Kanüle in eine Spritze, wird die DNA geschert. Diese Methoden sind neben ihrem hohen Arbeitsaufwand auch nicht geeignet, ein sauberes Arbeiten zu garantieren. So erweist sich der Homogenisierungsprozeß zur Isolierung von Biomolekülen aus potentiell infektiösem Ausgangsmaterial, wie menschliche Zell- und/oder Gewebeproben (Biopsiematerial), als gefährlichste Quelle für eine Kontamination des Versuchsdurchführenden, da das Homogenat bei der konventionellen Homogenisierung über eine beträchtliche Distanz spritzen kann.

Die Extraktion von Nucleinsäuren und anderen Biomolekülen aus pflanzlichen Materialien erweist sich aufgrund des hohen Gehalts an Polysacchariden, Polyolen und/oder anderen sekundären Metaboliten als äußerst schwierig, da die genannten Substanzen nach Aufschluß der Zellen oder Gewebe in den üblicherweise verwendeten Löschungen hochviskose Lysate oder gallertartige Strukturen ausbilden. Einfaches Abzentrifugieren der gallertartigen Masse führt in den meisten Fällen nicht zum Erfolg, da eine Trennung von der verwendeten Lösung nicht erreicht werden kann. Die gallertartigen Strukturen und die hohe Viskosität verhindern eine effiziente Isolierung von Nucleinsäuren bzw. machen sie teilweise unmöglich.

Auch spezielle Homogenisatoren wie die handelsüblichen Ultraturrax™, Polytron™, Omni™, Tissuemizer™ u.ä. können zur Scherung von hochmolekularer DNA eingesetzt werden. Diese Methode ermöglicht zwar ein einfaches und schnelles Homogenisieren praktisch jeder Probe, nachteilig ist allerdings, daß zur Homogenisierung sehr kleiner Zell-, Gewebe- oder Lösungsmengen, die insbesondere nachfolgend zur Analyse mittels PCR oder RT-PCR vorgesehen sind, spezielle Mini-Generatoren angeschafft werden müssen. Um Cross-Kontaminationen zwischen verschiedenen Zell- oder Gewebeproben oder anderen Proben zu vermeiden, können dann auch miniaturisierte Einmal-Generatoren verwendet werden, wobei jedoch vorausgesetzt werden muß, daß ein relativ aufwendiges Gerät zum Homogenisieren bereits vorhanden ist. Die miniaturisierten Einmal-Generatoren selbst sind darüber hinaus sehr kostenintensiv.

Eine weitere Methode zur Homogenisierung, die sich für einige Anwendungen bewährt hat, ist eine Ultraschallbehandlung der betreffenden Probe. Dabei ist auf dem angesprochenen Gebiet, beispielsweise der PCR-Anwendung, teures Gerät erforderlich. Nachteilig ist ebenfalls, daß Einmal-Ultraschallköpfe nicht erhältlich sind. Es besteht darüber hinaus die Gefahr, daß die Nucleinsäuren durch die Ultraschallbehandlung zu stark fragmentiert werden bis zur völligen Unbrauchbarkeit für die weiteren Analyse-Methoden.

Die DE-A-4 139 664 betrifft eine Vorrichtung und ein Verfahren zur Isolierung und Reinigung von Nucleinsäuren aus natürlichen Quellen durch Abtrennung der aufgeschlossenen natürlichen Quellen wie Zellen oder Telltrümmer in einer Probe durch Filtration. Die Vorrichtung zur Durchführung des beschriebenen Verfahrens besteht aus einem Hohlkörper mit einer Einlaßöffnung und einer Auslaßöffnung, wobei im Hohlkörper zwischen zwei Fixiereinrichtungen ein pulverförmiges erstes Material auf Silicagelbasis angeordnet ist und ein zweites Material zwischen dem ersten und der Auslaßöffnung angeordnet ist, wobei die ersten und zweiten Materialien unterschiedliche Adsorptionscharakteristika für Nucleinsäuren aufweisen.

DE-U-91 12 776 beschreibt ein Filtrationssystem zur Abtrennung von ungelösten Stoffen aus einem flüssigen Medium, bei dem die zu filtrierende Flüssigkeit parallel zur Oberfläche eines Filters geführt wird, dadurch gekennzeichnet, daß der Filter aus einer mehrschichtigen Gewebeanordnung besteht, bei der die Trenngrenzen der einzelnen Gewebeschichten in Richtung des Filtratstromes zunehmen.

Die DE-A-4 034 036 betrifft eine Vorrichtung und ein Verfahren zur Isolierung von Nucleinsäuren aus Zellsuspensionen. Die Vorrichtung zur Durchführung dieses Verfahrens weist eine zellenaufnehmende Matrix in einem Hohlkörper zwischen zwei porösen Einrichtungen auf. Die Porengröße der Einrichtungen ist größer als die Hohlraumgröße des die Matrix bildenden Materials.

CLONTECH Labs 1993, "Nucleic Acid Purification with CHROMA SPIN Columns", betrifft ein Verfahren zur Extraktion von Nucleinsäuren aus Agarosegelstücken.

In D. Blöcher und G. Iliakis, Int. J. Radiat. Biol., 1991, Vol. 59, 919 - 926, werden DNA-Fragmente von einem Filter entfernt während einer nicht denaturierenden Filterelution. Dabei scheinen DNA-Stücke an der Filtermembran abgebaut zu werden.

Das der Erfindung zugrundeliegende Problem besteht demnach darin, ein Verfahren anzugeben, das die genannten Nachteile des Standes der Technik vermeidet. Es soll ein Verfahren bereitgestellt werden, das es in einfacher und kostengünstiger Weise ermöglicht, eine effiziente und crosskontaminationsfreie Präparation von Nucleinsäuren und generell eine Homogenisierung von viskosen Systemen zu erreichen.

Das der Erfindung zugrundeliegende technische Problem wird gelöst durch ein Verfahren mit den Merkmalen des Anspruchs 1. Die sich daran anschließenden Unteransprüche 2 bis 6 betreffen bevorzugte Ausführungsformen des erfindungsgemäßen Verfahrens.

Der Anspruch 7 betrifft die Verwendung der erfindungsgemäßen Vorrichtung.

Das erfindungsgemäße Verfahren zur Zerkleinerung von hochmolekularen Strukturen insbesondere hochmolekularer Nucleinsäuren geht davon aus, daß das die zu zerkleinernden Strukturen enthaltende System auf einer Einrichtung angeordnet wird. Diese Einrichtung weist mindestens eine poröse Schicht auf. Die poröse Schicht weist dabei eine asymmetrische Verteilung ihrer Porengröße auf. Die Porengröße nimmt dabei in Passierrichtung der zu zerkleinernden Strukturen, durch die poröse Schicht gesehen, ab. Der Begriff Porengröße ist als mittlere Porengröße zu verstehen. Das die zu zerkleinernden hochmolekularen Strukturen enthaltende System passiert die Einrichtung, wobei während der Passage die hochmolekularen Strukturen, vermutlich aufgrund ihrer Empfindlichkeit gegen mechanische Einwirkungen, zerkleinert werden. Das die zu zerkleinernden hochmolekularen Strukturen enthaltende System kann insbesondere ein relativ hoch viskoses System sein, das aus einem Zell- und/oder Gewebelysat besteht oder eine zur Trennung von Nucleinsäuren verwendete Matrix, wie Polyacrylamidgele, oder Weichgewebe (Brustgewebe, Hirn, Fettgewebe u. ä.), oder Lösungen, die hochmolekulare DNA enthalten.

Das erfindungsgemäße Verfahren kann in vorteilhafter Weise auch zur Homogenisierung von inhomogenen viskosen Systemen, insbesondere Zell- und Gewebelysaten, verwendet werden, wenn die oben beschriebene Einrichtung mindestens zwei poröse Schichten aufweist. Das die zu zerkleinernden hochmolekularen Strukturen enthaltende oder zu homogenisierende viskose System passiert die Einrichtung mit mindestens zwei porösen Schichten, wobei die Porengröße der porösen Schicht in Passierrichtung des zu homogenisierenden Systems gesehen abnimmt. Die Porengröße der in Passierrichtung gesehenen ersten Schicht kann bis zum 6-fachen der Porengröße der darunterliegenden zweiten Schicht betragen. Die zweite Schicht besitzt eine Mindestporengröße, die im wesentlichen dadurch bestimmt ist, daß eine Verstopfung der zweiten Schicht durch die zu zerkleinernde oder homogenisierende Probe unterbleibt. Typischerweise ist die Porengröße der zweiten Schicht > 10 µm. Damit kann auch eine zur Trennung von Nucleinsäuren oder Proteine verwendete Matrix, wie Polyacrylamidgele, zerkleinert bzw. homogenisiert werden.

Der Vorgang kann mehrfach wiederholt werden, wenn sich zeigt, daß keine hinreichende Scherung nach einmaliger Passage erreicht wurde. Es versteht sich, daß das erfindungsgemäße Verfahren ebenso dahingehend abgewandelt werden kann, daß die Passierstrecke für die zu zerkleinernde Struktur verlängert wird, indem die Dicke der verwendeten porösen Schicht oder Schichten variiert wird.

Zellen- und/oder Gewebe können gegebenenfalls in Guanidinthiocyanat-haltigem Puffer oder in einem Proteinase K oder ähnliche Protease enthaltenden Puffer aufgenommen und/oder gegebenenfalls darin inkubiert werden. Die Zellen- oder Gewebe stücke können vorher durch mechanische Einwirkung zerkleinert worden sein.

Die erfindungsgemäße Vorrichtung ist zur Isolierung von Nucleinsäuren problemlos. Hochviskoses Material wird in dieser Vorrichtung geschert, so daß das Lysat anschließend einfach für die weitere Verarbeitung benutzt werden kann, während Zell- und Gewebereste gleichzeitig aus dem Lysat durch den Filtrationseffekt der Vorrichtung entfernt werden.

Die Passage des viskosen Systems durch die Poren bewirkt überraschenderweise eine Scherung von gegen mechanische Einflüsse empfindlichen Substanzen. Insbesondere wird durch die Passage hochmolekulare Nucleinsäure, wie genomische DNA geschert. Gleichzeitig können durch das erfindungsgemäße Verfahren genomische DNA oder andere hochmolekulare Verbindungen enthaltende Lösungen homogenisiert und hochmolekulare Strukturen wie Polyacrylamidgele zerkleinert werden. Nach der Homogenisierung weisen die entsprechenden Proben eine geringere Viskosität als zu Anfang der Behandlung auf. Die Proben können dementsprechend leichter weiterverarbeitet werden.

Das erfindungsgemäße Verfahren ermöglicht die Probenaufbereitung zur Isolierung von RNA und DNA. Dabei werden RNA-Moleküle, insbesondere in einem Größenbereich von 0,8 bis 20 Kbp zugänglich. DNA-Moleküle in einer Größe von 40.000 bis 50.000 bp werden aus Proben erhalten, die DNA als chromosomale DNA enthalten.

In einer bevorzugten Ausgestaltung des erfindungsgemäßen Verfahrens wird das die zu zerkleinernde hochmolekulare Struktur aufweisende System oder das zu homogenisierende System (Probe) auf der Einrichtung mit mindestens einer porösen Schicht angeordnet und auf der Probenseite unter mechanischer Einwirkung durch die mindestens eine poröse Schicht gedrückt. Die Passage kann beispielsweise auf der Probenseite mit mechanischer Einwirkung wie Überdruck oder Schwerkraft, wie sie durch Zentrifugation generiert werden kann, bewirkt werden. Es ist jedoch ebenfalls möglich, auf der der Probe abgewandten Seite einen Unterdruck zu erzeugen, wodurch dann z.B. das zu homogenisierende viskose System die porösen Schichten der Einrichtung passiert.

Die erfindungsgemäße Vorrichtung erlaubt in vorteilhafter Weise eine Probenvorbereitung für die PCR-Reaktion (PCR: Polymerase Chain Reaction) . Diese Reaktion dient der DNA-Amplifikation. Insbesondere bei Verwendung einer Schicht mit einer Porengröße im Bereich von 20 µm bis 70 µm fällt die DNA überwiegend in Längen von 40 bis 60 kb an, unabhängig davon, ob die DNA direkt aus Zell-Lysaten stammt oder aus vorgereinigten Lösungen. Somit läßt sich die Erfindung in vorteilhafter Weise mit dem in DE 43 21 904 A1 beschriebenen Verfahren kombinieren. Auf der porösen Schicht wird eine Nucleinsäure bindende Matrix wie z.B. Glasfasern, angeordnet. Die vorhandene DNA wird dabei unter hohen Ionenstärken auf der Matrix gebunden und bei Elution, mit z.B. Wasser, danach durch Passieren der poröse(n) Schicht(en) geschert (zerkleinert).

Analog ist auch bei der einschichtigen Ausführungsform des Verfahrens vorzugehen.

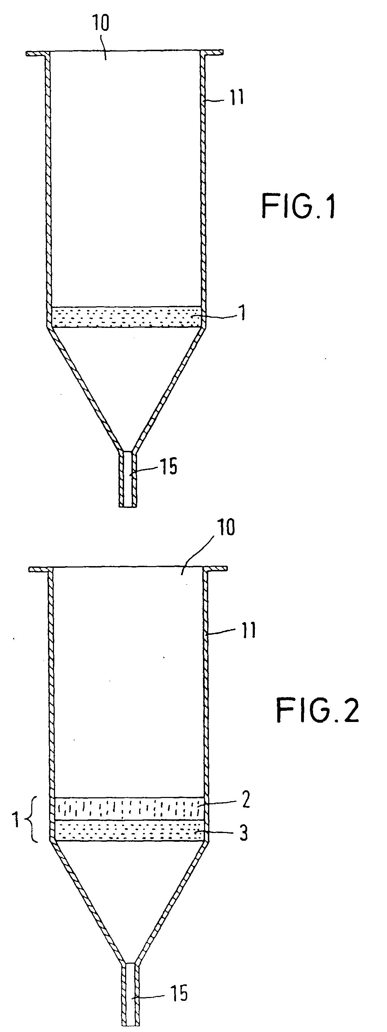

Die Figur 1 zeigt eine an das erfindungsgemäße Verfahren angepaßte Vorrichtung, die ebenfalls gemäß Anspruch 7 Gegenstand der vorliegenden Erfindung ist. Die Vorrichtung weist eine Einlaß- 10 und Auslaßöffnung 15, mit einer im Lumen eines Hohlkörpers 11 angeordneten Einrichtung 1 auf. Die Einrichtung 1 besteht aus einer porösen Schicht, deren Porengröße, in Passierrichtung der zu zerkleinernden Strukturen durch die poröse Schicht gesehen, abnimmt. Die poröse Schicht besteht vorzugsweise aus inerten Materialien, wie anorganischen oder organischen Substanzen, insbesondere Glasfritten-ähnliche Materialien oder polymere Substanzen, wie sie auch zum Aufbau von Membranfiltern verwendet werden. Es sind insbesondere Polyethylen, Polypropylen, Polystyrol und andere polymere Kohlenwasserstoffmaterialien zu nennen, aus denen die poröse Schicht bestehen kann.

Die Figur 2 zeigt eine an das erfindungsgemäße Verfahren angepaßte Vorrichtung, die ebenfalls gemäß Anspruch 8 Gegenstand der vorliegenden Erfindung ist. Die weist eine Einlaß- 10 und Auslaßöffnung 15, mit einer im Lumen eines Hohlkörpers 11 angeordneten Einrichtung 1 auf. Die Einrichtung 1 besteht aus mindestens zwei Schichten 2,3 mit in Richtung der Auslaßöffnung 15 abnehmender Porengröße der Schichten 2,3. Durch die Passage des zu homogenisierenden Systems durch die Schichten 2,3 wird dieses homogenisiert. Die Schichten sind vorzugsweise in dem insbesondere zylindrisch ausgestalteten Hohlkörper 11 befestigt. Diese Befestigung kann durch Reibung, wie beispielsweise durch Einspannen der Einrichtung 1 mit den Schichten 2,3 oder durch Verkleben mit der insbesondere zylindrischen Hohlraumwandung erfolgen. Die Schichten weisen Porengröße im Bereich von 1 mm bis 10 µm auf, wobei die Porengröße der jeweiligen Schichten abgestuft ist.

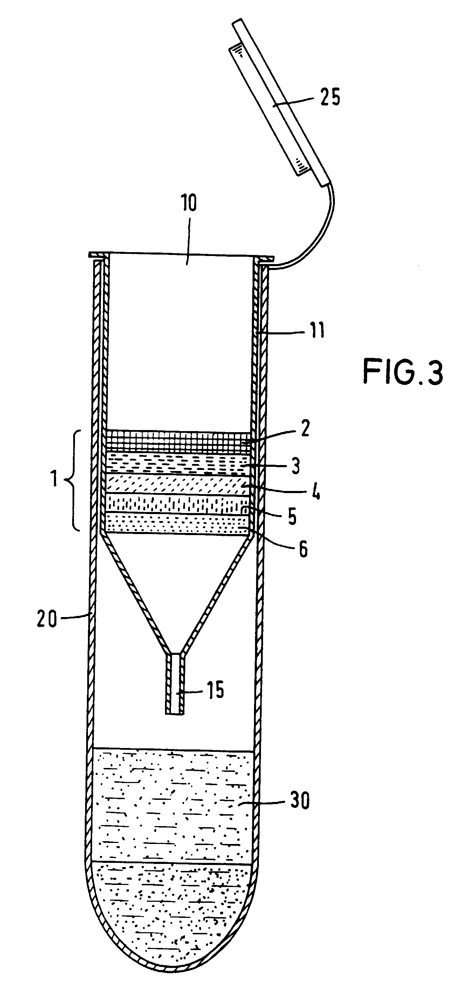

Die Abstufung der Porengröße der Schichten erfolgt dadurch, daß die erste Schicht 2, in Fließrichtung der Probe gesehen, eine bis zum 6-fachen der Porengröße der zweiten Schicht 3 aufweist, wobei deren Porengröße > 10 µm ist. In einer besonderen Anordnung ist die erfindungsgemäße Vorrichtung auf handelsübliche Zentrifugenröhrchen abgestimmt. Die Figur 3 zeigt eine solche Anordnung. Dabei ist ein im wesentlichen zylindrischer Hohlkörper mit an der Auslaßöffnung 15 trichterförmig verjüngend am oberen Ende des Zentrifugenröhrchens so angeordnet, daß die Einlaßöffnung 10 der erfindungsgemäß zu verwendenden Vorrichtung ebenfalls an der Öffnung des Zentrifugenröhrchens 20 angeordnet ist. Die Anordnung kann mittels eines Deckels 25, der entweder an der erfindungsgemäßen Vorrichtung oder an dem Zentrifugenröhrchen selbst angeordnet ist, verschlossen werden. Im Innern der erfindungsgemäßen Vorrichtung sind fünf Schichten 2,3,4,5,6 vorzugsweise so angeordnet, daß die Schicht mit der jeweils geringeren Porengröße direkt an diejenige mit der größeren Porengröße mit ihren Oberflächen aneinandergrenzt. Nach Passage des viskosen Systems durch die Einrichtung 1 sammelt sich am Boden des Zentrifugenröhrchens 20 die homogenisierte Lösung 30.

In der in Figur 3 gezeigten Anordnung weisen die porösen Schichten beginnend mit der Schicht 2 Porengrößen von ca. 200 µm, Schicht 3 ca. 75 µm, Schicht 4 ca. 35 µm, Schicht 5 ca. 20 µm und Schicht 6 ca. 10 µm auf. Es versteht sich, daß die Vorrichtung auch mit nur einer porösen Schicht, wie oben beschrieben, eingesetzt werden kann.

Es ist für den Fachmann selbstverständlich, daß eine einstückig ausgeformte Einrichtung, die nicht aus mehreren Lagen aufgebaut ist, als äquivalente Ausführungsform verstanden wird. Wichtig ist dann, daß die in einer einstückigen Version ausgeführte Einrichtung 1 einen Porengrößengradienten, der von einer Oberfläche der Schicht zu der gegenüberliegenden Schicht verläuft, aufweist. Solche Vorrichtungen sind aus DE 41 39 664 A1 bekannt.

Die erfindungsgemäßen Verwendungen der beschriebenen Vorrichtungen erlauben, daß in einfacher, kostengünstiger Weise viskose Systeme wie beispielsweise Zell- und Gewebelysate homogenisiert werden und in Systeme geringerer Viskosität überführt werden können.

Es hat sich überraschenderweise gezeigt, daß hochmolekulare Nucleinsäuren aufgrund der Passage durch die in der erfindungsgemäß zu verwendenden Vorrichtung befindlichen Einrichtung geschert wird, so daß sie für die nachfolgenden molekularbiologischen Prozessierungsschritte leichter und reproduzierbarer weiterverarbeitbar wird. Dies trifft insbesondere für die Aufarbeitung von genomischer DNA, mRNA und Gesamt-RNA/-DNA zu.

Weiterhin hat sich überraschenderweise gezeigt, daß hochmolekulare Strukturen wie Polyacrylamidgele in einfacher Weise zerkleinert werden können, so daß z.B. Nucleinsäuren und/oder Proteine leicht aus einer Trennmatrix isoliert werden können.

In einer anderen Weiterbildung der erfindungsgemäßen Vorrichtung ist die Einrichtung 1 in einer Spritze (als zylindrischer Hohlkörper 5) angeordnet. Das zu homogenisierende viskose System wird dabei durch die Kanüle in die Spritze gezogen und passiert die in der Nähe der Verbindungsstelle zwischen Kanüle und Spritze angeordnete Einrichtung 1. Ist die Probe in die Spritze gezogen, kann diese durch Herausdrücken erneut die Einrichtung 1 passieren. Gegebenenfalls kann dieser Vorgang mehrfach wiederholt werden, um eine hinreichende Homogenisierung, insbesondere hochviskoser Lösungen enthaltend hochmolekulare Nucleinsäuren, zu gewährleisten. Dabei kann die Schicht mit der größten Porengröße der Kanüle der Spritze zugewandt sein.

In vorteilhafter Weise können durch das erfindungsgemäße Verfahren und/oder die erfindungsgemäße Vorrichtung Nucleinsäuren, wie genomische DNA oder Gesamt-Nucleinsäuren aus den entsprechenden Matrices isoliert werden, die üblicherweise zur Trennung von Nucleinsäuren eingesetzt werden. Zu nennen sind hierbei insbesondere Polyacrylamidgele, die als Matrix für gelelektrophoretische Trennungen von DNA oder RNA eingesetzt werden. Nach der Elektrophorese werden lediglich die Banden, in welcher sich die Nucleinsäuren bestimmter Größe angereichert haben, beispielsweise durch Stanzen oder Schneiden von den übrigen Gelbestandteilen getrennt und dann durch die erfindungsgemäß zu verwendende Vorrichtung unter Anwendung des erfindungsgemäß beschriebenen Verfahrens passiert. Dabei hat sich überraschenderweise gezeigt, daß die in der homogenisierten Lösung vorhandenen Restbestände des Gels keine störenden Einflüsse auf die folgenden molekularbiologischen Prozessierungsschritte der Nucleinsäuren zeigt.

Die Erfindung beschreibt somit ein einfaches, sicheres und kostengünstiges Verfahren zur Zerkleinerung und Homogenisierung von viskosen Systemen, insbesondere Zell- und Gewebelysaten oder hochmolekulare Nukleinsäuren enthaltenden Systemen.

Die vorliegende Erfindung wird anhand folgender Beispiele näher erläutert.

### Beispiele

Zusammensetzung der in den Beispielen 1 - 3 benutzten Lösungen
- OL:: 4 M Guanidin-Isothiocyanat, 25 mM Na-Citrat pH 7.0, 20 mM EDTA pH 8.0
- DB:: 25 mM Na-Citrat pH 7.0, 0,20 EDTA pH 8.0, 0,75 % Sarcosin
- OW:: 150 mM NaCl, 1 mM EDTA pH 8.0, 10 mM Tris-HCl pH 7.5
- TE:: 10 mM Tris-HCl pH 7.5, 1 mM EDTA pH 8.0

### Beispiel 1

### Homogenisierung von Zell-Lysaten und anschließende direkte Isolierung von poly A⁺ mRNA aus HeLa-Zellen.

HeLa-Zellen, die auf einer 10 cm Zellkulturschale bis zu konfluentem Wachstum (ca. 1 x 10⁷ Zellen) herangezogen worden sind, wurden in 600 µl OL direkt auf der Zellkulturschale lysiert. Das Lysat wurde mit einem Zellschaber auf einer Seite der Schale gesammelt, und auf eine Vorrichtung gemäß der vorliegenden Patentanmeldung pipettiert. Im Inneren der Vorrichtung wurden zwei Schichten aus CELLPOR™ PE Filterplatten von 200 µm und 35 µm Porengröße so angeordnet, daß sich die Schicht mit der geringeren Porengröße in Richtung der Auslaßöffnung befand. Die Vorrichtung wurde in einem 2 ml Mikrozentrifugengefäß plaziert. Anschließend wurde das viskose Lysat durch die Vorrichtung 3 min bei voller Geschwindigkeit in einer herkömmlichen Tischzentrifuge (ca. 14.000 bis 18.000 g) zentrifugiert. Das so erhaltene nicht-viskose Lysat wurde mit 1.200 µl DB versetzt und 3 min zentrifugiert. Der Überstand wurde in ein neues Zentrifugenröhrchen überführt, mit 35 µl, einer Oligotex Suspension versetzt und für 10 min inkubiert. Anschließen wurden die mRNA: Oligotex Komplexe pelletiert, und zweimal in 600 µl OW durch Resuspendieren und Zentrifugieren gewaschen. Die mRNA wurde dann in Wasser von den Oligotex-Partikeln eluiert, und nach Zentrifugation und Pelletierung der Latexpartikel als gereinigte mRNA-Fraktion in ein neues Mikrozentrifugengefäß überführt.

### Beispiel 2

### Steigerung der Amplifikationseffizienz in der PCR-Reaktion durch Scherung der DNA

Genomische DNA mit einer durchschnittlichen Größe von 100 kb wurde aus HeLa-Zellen gemäß der Prozedur in "Current Protocols in Molecular Biology" (Ausubel et al., Wiley and Sons, Boston, Vol. 1, Seiten 2.2.1 bis 2.2.3) isoliert. In Parallelansätzen wurde die DNA in Tris-Puffer über eine Vorrichtung gemäß der vorliegenden Patentanmeldung mit jeweils einer Schicht aus CELLPOR™ PE Filterplatten von 200 µm, 50 µm, 35 µm und 5 µm Porengröße 2 min zentrifugiert. Im PCR-Ansatz wurden anschließend jeweils 1 µg der unterschiedlich gescherten DNA sowie nicht-gescherte DNA eingesetzt, um ein 140 bp Fragment des Globin-Gens zu amplifizieren. In der PCR-Reaktion wurden, bei gleicher Anzahl an Zyklen, mit gescherter DNA größere Ausbeute an Amplifikat erzielt als mit ungescherter DNA.

### Beispiel 3

### Isolierung von RNA aus einem Polyacrylamid-Gelstück nach Zerkleinerung des Polyacrylamidgels

Auf einem denaturierenden 5 %igen Polyacrylamid-Harnstoffgel wurde in vitro transkribiert RNA aufgetragen, um das "full-length" Transkript von kürzeren Transkripten zu trennen. Nach Ethidiumbromid Färbung der RNA wurde die entsprechende, 360 Basen große Bande ausgeschnitten, und auf eine Vorrichtung gemäß der vorliegenden Patentanmeldung mit einer Schicht aus CELLPOR PE Filterplatten von 200 µm überführt. Dann wurden 500 µl TE-Puffer auf die Vorrichtung pipettiert. Anschließend wurde für 3 min bei voller Geschwindigkeit in einer herkömmlichen Tischzentrifuge (ca. 14.000 bis 18.000 g) zentrifugiert. Das so zerkleinerte Gelstückchen wurde erneut auf eine Vorrichtung mit einer Schicht aus einer CELLPOR™ PE Filterplatte mit 1 µm Porengröße überführt, um die kleinen Polyacrylamid-Harnstoffgelstücke von der Flüssigkeit zu trennen. Die wäßrige Fraktion im Auffanggefäß wurde dann Phenol/ Chloroform extrahiert. Die obere, wäßrige Phase wurde abpipettiert, und die darin gelöste RNA mit LiCl (Endkonzentration 3 M) 3 Stunden bei 20°C gefällt. Das RNA-Pellet wurde zweimal mit 70 % Alkohol gewaschen, abzentrifugiert und unter Vakuum getrocknet. Anschließend wurde die RNA in TE gelöst.

### Beispiel 4

### Isolierung von RNA aus Weichgewebe nach Homogenisierung des Gewebes über die erfindungsgemäße Vorrichtung.

Zusammensetzungen der in den Beispielen 4 und 5 benutzten Lösungen
- R1:: 3,5 M Guanidin-Isothiocyanat, 25 mM Na-Citrat pH 7,0, 1% β-Mercaptoethanol
- R2:: 900 mM GTC, 25 mM Tris-HCl pH 7,5, 10% Ethanol
- TE:: 50 mM Tris-HCl pH 7,5, 80% Ethanol

20 mg Brustgewebe wurden auf eine erfindungsgemäße Vorrichtung gelegt und 350 µl Lysepuffer R1 dazu pipettiert. Im Inneren der Vorrichtung wurden 2 Schichten aus CELLPOR PE Filterplatten von 200 µm und 50 µm Porengröße so angeordnet, daß sich die Schicht mit der geringeren Porengröße in Richtung der Auslaßöffnung befand. Die Vorrichtung wurde in einem 2 ml Mikrozentrifugengefäß plaziert. Anschließend wurde das viskose Lysat durch die Vorrichtung 3 min bei voller Geschwindigkeit in einer herkömmlichen Tischzentrifuge (ca. 14.000 bis 18.000 g) zentrifugiert. Das so erhaltene nicht-viskose Lysat wurde mit 350 µl 70% Ethanol versetzt und gemäß P 44 04 361 auf eine RNeasy™ Spin Säule, die in einem 2 ml Mikrozentrifugengefäß hängt, pipettiert. Anschließend erfolgt eine Zentrifugation bei 8.000 x g für 15 sec in einer Standardtischzentrifuge. Die RNeasy™ Spin Säule wird in ein neues 2 ml Gefäß transferiert, und mit 700 µl Waschpuffer R2 gewaschen (15 sec Zentrifugation bei 8.000 x g) . Daraufhin wird die Säule gleichermaßen zweimal mit 500 µl Waschpuffer TE gewaschen. Zur Elution der RNA wird die Säule in ein neues 1,5 ml Reaktionsgefäß gesetzt, 50 µl DEPC-behandeltes Wasser werden direkt auf die Membran der Spinsäule pipettiert, und die RNA anschließend durch 1 min Zentrifugation bei 8.000 x g aufgefangen.

### Beispiel 5

### Isolierung von RNA aus pflanzlichen Zellen und Geweben nach Zentrifugation über die erfindungsgemäße Vorrichtung.

100 mg Blattgewebe von Pelagonien wurden unter flüssigem Stickstoff zu einem feinen Pulver verrieben. Anschließend wurde das Pulver in 450 µl Lysepuffer R1 lysiert und das so erhaltene Lysat auf die erfindungsgemäße Vorrichtung pipettiert. Im Inneren der Vorrichtung wurden 2 Schichten aus CELLPOR PE Filterplatten von 200 µm und 50 µm Porengröße so angeordnet, daß sich die Schicht mit der geringeren Porengröße in Richtung der Auslaßöffnung befand. Die Vorrichtung wurde in einem 2 ml Mikrozentrifugengefäß plaziert. Anschließend wurde das viskose Lysat durch die Vorrichtung 3 min bei voller Geschwindigkeit in einer herkömmlichen Tischzentrifuge (ca. 14.000 bis 18.000 g) zentrifugiert. Das so erhaltene nicht-viskose Lysat wurde mit 225 µl 100% Ethanol versetzt und gemäß P 44 04 361 auf eine RNeasy™ Spin Säule, die in einem 2 ml Mikrozentrifugengefäß hängt, pipettiert. Anschließend erfolgt eine Zentrifugation bei 8.000 x g für 15 sec in einer Standardtischzentrifuge. Die RNeasy™ Spin Säule wird in ein neues 2 ml Gefäß transferiert, und mit 700 µl Waschpuffer R2 gewaschen (15 sec Zentrifugation bei 8000 x g) . Daraufhin wird die Säule gleichermaßen zweimal mit 500 µl Waschpuffer TE gewaschen. Zur Elution der RNA wird die Säule in ein neues 1,5 ml Reaktionsgefäß gesetzt, 50 µl DEPC-behandeltes Wasser werden direkt auf die Membran der Spinsäule pipetiert, und die RNA anschließend durch 1 min Zentrifugation bei 8.000 x g aufgefangen.

## Patentansprüche

1. Verfahren zum Zerkleinern hochmolekularer Strukturen, insbesondere hochmolekularer Nucleinsäurestrukturen in Proben, wobei die zu zerkleinernden hochmolekularen Strukturen eine Einrichtung passieren, die mit mindestens einer porösen Schicht versehen ist, deren Porengröße in Passierrichtung der zu zerkleinernden Strukturen, durch die poröse Schicht gesehen, abnimmt.

2. Verfahren nach Anspruch 1, wobei die poröse Schicht aus mindestens zwei Schichten aufgebaut wird, deren mittlere Porengröße unterschiedlich ist, wobei die in Fließrichtung der Proben gesehene erste Schicht eine mittlere Porengröße bis zu dem 6-fachen der Porengröße der zweiten Schicht aufweisen kann und die zweite Schicht eine mittlere Porengröße größer als 10 µm besitzt.

3. Verfahren nach Anspruch 1 und/oder 2, wobei die zu zerkleinernden hochmolekularen Strukturen eine viskose Lösung sind.

4. Verfahren nach Anspruch 3, wobei die viskose Lösung ein Zell- und/oder Gewebelysat oder eine zur Trennung von Nucleinsäuren verwendete Matrix, wie Polyacrylamidgele ist.

5. Verfahren nach mindestens einem der Ansprüche 1 bis 4 zur Scherung von Nucleinsäuren wie genomischer DNA, zur Homogenisierung genomischer DNA oder anderer hochmokulare Verbindungen enthaltender Lösungen.

6. Verfahren nach mindestens einem der Ansprüche 1 bis 5, wobei das zu zerkleinernde System (Probe) auf der Einrichtung mit poröser Schicht angeordnet wird und auf der Probenseite mit mechanischer Einwirkung, wie Überdruck oder erhöhter Schwerkraft und/oder auf der probenabgewandten Seite durch Unterdruck, die Schicht der Einrichtung passiert.

7. Verwendung einer Vorrichtung mit einer Einlaß- (10) und Auslaßöffnung (15), mit mindestens einer im Lumen eines Hohlkörpers (11) angeordneten Einrichtung aus einer Schicht (1) zur Zerkleinerung hochmolekularer Strukturen, wobei die mittlere Porengröße der Schicht (1) in Passierrichtung der zu zerkleinernden Strukturen, durch die poröse Schicht (1) gesehen, abnimmt zur
- Homogenisierung eines viskosen Systems wie Zell- oder Gewebelysaten,
- Homogenisierung von genomischer DNA,
- Zerkleinerung (Scherung) von genomischer DNA,
- Zerkleinerung von Polyacrylamidgelen,
- Probenvorbereitung durch Zerkleinern für die Isolierung von mRNA und Gesamt-RNA,
- Probenvorbereitung durch Zerkleinern für die Isolierung von mRNA und Gesamt-RNA und DNA aus pflanzlichen Zell- und Gewebelysaten.

## Claims

1. A method for the size reduction of high-molecular structures, in particular high-molecular nucleic acid structures, in samples wherein the high-molecular structures to be size-reduced are passed through a means provided with at least one porous layer the pore size of which decreases in the direction of the passage of the structures to be size-reduced through the porous layer.

2. The method according to claim 1, wherein said porous layer is composed of at least two layers with different average pore sizes wherein the first layer, as seen in the direction of sample flow, may have an average pore size which is up to six times the pore size of the second layer and the second layer has an average pore size of larger than 10 µm.

3. The method according to claims 1 and/or 2, wherein the high-molecular structures to be size-reduced are a viscous solution.

4. The method according to claim 3, wherein said viscous solution is a cell and/or tissue lysate, or a matrix used for the separation of nucleic acids, such as polyacrylamide gels.

5. The method according to at least one of claims 1 to 4 for the shearing of nucleic acids, such as genomic DNA, for the homogenization of solutions containing genomic DNA or other high-molecular compounds.

6. The method according to at least one of claims 1 to 5, wherein the system to be size-reduced (sample) is charged onto the means having a porous layer and is passed through the layer by mechanical action, such as increased pressure or increased gravity, at the sample side and/or by reduced pressure at the side opposite to the sample.

7. Use of a device having an inlet (10) and outlet (15), and at least one means arranged within the lumen of a hollow body (11) consisting of a layer (1) for the size-reduction of high-molecular structures wherein the average pore size of layer (1) decreases in the direction of the passage of the structures to be size-reduced through the porous layer (1), for the
- homogenization of a viscous system, such as cell or tissue lysates;
- homogenization of genomic DNA;
- size reduction (shearing) of genomic DNA;
- size-reduction of polyacrylamide gels;
- sample preparation by size-reduction for the isolation of mRNA and whole RNA;
- sample preparation by size-reduction for the isolation of mRNA and whole RNA and DNA from plant cell and tissue lysates.

## Revendications

1. Procédé pour fragmenter des structures à grande masse moléculaire, en particulier des structures d'acides nucléiques à grande masse moléculaire dans des échantillons, où les structures à grande masse moléculaire à fragmenter passent dans un dispositif qui est pourvu d'au moins une couche poreuse, dont le diamètre des pores diminue dans la direction de passage des structures à fragmenter, quand on regarde à travers la couche poreuse.

2. Procédé selon la revendication 1, dans lequel la couche poreuse est constituée d'au moins deux couches, dont le diamètre moyen des pores est différent, la première couche, quand on regarde dans la direction d'écoulement des échantillons, pouvant avoir un diamètre moyen des pores allant jusqu'à 6 fois le diamètre des pores de la deuxième couche, et la deuxième couche ayant un diamètre moyen des pores supérieur à 10 µm.

3. Procédé selon la revendication 1 et/ou 2, dans lequel les structures moléculaires à grande masse moléculaire à fragmenter sont une solution visqueuse.

4. Procédé selon la revendication 3, dans lequel la solution visqueuse est un lysat cellulaire et/ou tissulaire, ou encore une matrice utilisée pour séparer les acides nucléiques, telle que des gels de polyacrylamide.

5. Procédé selon au moins l'une des revendications 1 à 4 pour cisailler des acides nucléiques tels que l'ADN génomique, pour homogénéiser l'ADN génomique ou d'autres solutions contenant des composés à grande masse moléculaire.

6. Procédé selon au moins l'une des revendications 1 à 5, dans lequel le système à fragmenter (échantillon) est disposé sur le dispositif comportant une couche poreuse, et, sur le côté échantillon, et à l'aide d'une action mécanique, telle qu'une surpression ou une augmentation de la force de gravité, et/ou sur le côté opposé à l'échantillon, sous l'effet d'une dépression, traverse la couche du dispositif.

7. Utilisation d'un appareil comportant une ouverture d'entrée (10) et une ouverture de sortie (15), avec au moins un dispositif, disposé dans la lumière d'un corps creux (11), constitué d'une couche (1), pour fragmenter des structures à grande masse moléculaire, où le diamètre moyen des pores de la couche (1) diminue dans la direction de passage des structures à fragmenter, quand on regarde la couche poreuse (1), pour :
- homogénéiser un système visqueux, tel que des lysats cellulaires ou tissulaires,
- homogénéiser un ADN génomique,
- fragmenter (cisailler) un ADN génomique,
- fragmenter des gels de polyacrylamide,
- préparer des échantillons par fragmentation pour isolement de l'ARNm et de l'ARN total,
- préparer des échantillons par fragmentation pour isolement de l'ARNm et de l'ARN total, et de l'ADN provenant de lysats cellulaires et tissulaires végétaux.
